# EUROPEAN PATENT APPLICATION

(11) **EP 4 553 162 A1**
(43) Date of publication of application: **14.05.2025**
(21) Application number: 24828718.7
(22) Date of filing: 19.06.2024
(51) Int. Cl.: C12N 15/81, C12N 1/19, C07K 14/78, C12R 1/84

(54) **BIOLOGICAL METHOD FOR LARGE-SCALE PREPARATION OF COLLAGEN HAVING 164.88° TRIPLE-HELIX STRUCTURE**

(30) Priority: 28.09.2023 CN 202311272338
(71) Applicant: Shanxi Jinbo Bio-Pharmaceutical Co., Ltd., Shanxi 030032 (CN)
(72) Inventor: WANG, Lingling, Taiyuan, Shanxi 030032 (CN); LAN, Xiaobin, Taiyuan, Shanxi 030032 (CN); LIU, Zengyao, Taiyuan, Shanxi 030032 (CN); DENG, Pengjun, Taiyuan, Shanxi 030032 (CN); ZHANG, Yongjian, Taiyuan, Shanxi 030032 (CN); LIU, Xin, Taiyuan, Shanxi 030032 (CN); ZHANG, Xiaobo, Taiyuan, Shanxi 030032 (CN); WANG, Ying, Taiyuan, Shanxi 030032 (CN); YANG, Xia, Taiyuan, Shanxi 030032 (CN); HE, Zhenrui, Taiyuan, Shanxi 030032 (CN)
(74) Representative: Cabinet Becker et Associés
(86) International application number: PCT/CN2024/100187
(87) International publication number: WO 2025/066310

(57) **Abstract**

Provided is a large-scale biological method for preparing collagen having 164.88° triple helix structure. Provided is a method for constructing a yeast cell expressing collagen, comprising introducing a recombinant expression vector into the yeast cell, wherein the recombinant expression vector comprises a polynucleotide encoding the collagen, wherein the collagen comprises repeating units of the sequence as shown in SEQ ID No. 1, the number of the repeating units is 2-32, and the repeating units are directly linked. The collagen is recombinant type III humanized collagen, preferably collagen having a 164.88° triple helix structure. The present disclosure achieves large-scale production of a recombinant humanized collagen which has a 164.88° triple helix structure and belongs to type A by engineering a "chassis strain", and can meet the market demand through large-scale production.

## Description

This application claims the priority and benefits of the Chinese patent application No: 202311272338.8 filed on September 28, 2023 and titled "Biological method for large-scale preparation of collagen having a 164.88° triple helix structure".

### Technical field

The present disclosure relates to a method for preparing collagen having a 164.88° triple helix structure by large-scale biological fermentation and belongs to the field of synthetic biology technology.

### Background of the invention

There are 28 different collagens in humans. Collagen is the most abundant protein in humans and animals, accounting for 25-30% of the total proteins in the body and about 6% of body weight. Collagen is an important structural protein present in skin, bones, teeth, cornea, tendons, ligaments and various tissues and organs, which plays a role in supporting and protecting tissues of an organism. Collagen is the main component of the extracellular matrix, and its biological activities are mainly manifested in functions such as promoting cell adhesion, growth and differentiation, a response to cell signaling pathway, platelet aggregation, maintenance of cell and tissue organ function, and damage repair. Collagen exhibits a triple helical winding structure in spatial structure, and three independent collagen α peptide chains are mainly wound in the form of hydrogen bonds to form a helical structure. In the body, collagen exerts its biological effects in combine with tissues and cells in a triple helix structure. Therefore, the biological activities of collagen depend largely on its natural triple helix structure. The excellent biological activities of collagen make it have great application value and potential in biomedical materials, medical beauty, cosmetics and other fields.

The main collagen products on the market now are obtained by extraction from animal tissue. This method destroys the natural triple helix structure during the extraction process, affecting collagen to exert the biological activities. Moreover, collagen obtained by an extraction process from animal sources is uneven in term of molecular weights of peptides and the processability is poor. Due to the differences in amino acid sequences between animal collagen and human collagen, it may cause potential immunogenicity in clinical practice and has poor safety. Moreover, it may be necessary to solve the safety risks caused by animal viruses before collagen can be released to the market due to impact of the sources from which animal tissues are obtained. As the market requirements for the safety and effectiveness of collagen products increase, the traditional method of extracting collagen from animal tissues can no longer meet the demand of the existing market. Moreover, the demand for products using collagen as a biomaterial for medical scenarios is also increasing, and other ways are needed to solve the problems currently existing in the market. The production of recombinant collagen with a natural triple helix structure through molecular biology techniques such as genetic engineering has great potential in solving the above-mentioned problems.

There are already type III collagen products on the market that are prepared through genetic recombination by several companies, but they have difficulty to achieve a triple helix structure. The use of synthetic biological fermentation to produce collagen with a correct triple helix structure has high technical barriers.

There is a need in this field for a method of producing collagen with a correct triple helix structure through synthetic biological fermentation.

### Summary of the invention

The inventors have discovered a human type III collagen, and structural studies have confirmed that the core functional region of the human type III collagen has a 164.88° triple helix structure (CN109593126A). The inventors have successfully prepared a recombinant type III humanized collagen using an *Escherichia coli* expression system by genetic recombination. However, the inventors found that the *E. coli* expression system has the disadvantages of limited protein expression and limited process amplification. Therefore, the inventors tried to express the human type III collagen in a eukaryotic expression system. After extensive preliminary screening work, the inventors found that when the human type III collagen is expressed in *Pichia pastoris,* a large amount of collagen with a 164.88° triple helix structure can be synthesized *in vitro.* In the previous application, the inventors also found that the human type III collagen has bacterial endotoxins when expressed in *E. coli,* which increases the complexity and difficulty of collagen purification. The inventors eliminate this technical problem by expressing collagen in a yeast expression system.

The present disclosure achieves large-scale production of recombinant humanized collagen having a 164.88° triple helix structure and which belongs to type A by engineering a "chassis strain", and can meet market demand through large-scale production.

In one aspect, a method for constructing a yeast cell expressing collagen is provided, comprising introducing a recombinant expression vector into a yeast cell, wherein the recombinant expression vector comprises a polynucleotide encoding collagen, and the collagen comprises repeating units of the sequence as shown in SEQ ID No. 1, the number of repeating units is 2-32, and the repeating units are directly linked. In one embodiment, the collagen is recombinant type III humanized collagen. In one embodiment, the collagen is collagen having a 164.88° triple helix structure.

In one embodiment, the collagen comprises the amino acid sequence shown in SEQ ID No. 2 or 3. In one embodiment, the polynucleotide comprises the nucleotide sequence as shown in SEQ ID No. 4 or 5. The polynucleotide of the present disclosure can effectively express collagen in a yeast host cell.

In one embodiment, the recombinant expression vector is a yeast expression vector. In one embodiment, the yeast expression vector is pPICZα A, pPIC9, pPIC9K, pHIL-S1, or pYAM75P vector.

In one embodiment, the yeast is *Pichia pastoris.* In one embodiment, the *P*. *pastoris* is *P. pastoris* X33, GS115, SMD1168, KM71, or KM71H strain.

In one embodiment, the method comprises one or more of the following steps:
(1) inserting a polynucleotide into an expression vector, preferably between NotI and XhoI restriction sites of pPiczalαA expression vector, to obtain a recombinant expression plasmid;
(2) enzymatically digesting the recombinant expression plasmid, preferably with sac1;
(3) purifying the enzymatically digested recombinant expression plasmid;
(4) introducing the enzymatically digested recombinant expression plasmid into a yeast cell by electroporation.

In another aspect, a yeast cell expressing collagen is provided, wherein the collagen comprises repeating units of a sequence as shown in SEQ ID No. 1, the number of repeating units is 2-32, and the repeating units are directly linked; the yeast cell is *P. pastoris.*

In one embodiment, *P. pastoris* is *P. pastoris* X33, GS115, SMD1168, KM71, or KM71H strain. In one embodiment, the collagen comprises the amino acid sequence shown in SEQ ID No. 2 or 3. In one embodiment, the collagen is a recombinant type III humanized collagen. In one embodiment, the collagen is collagen having a 164.88° triple helix structure.

In one embodiment, the yeast cell comprises a yeast expression vector. In one embodiment, the yeast expression vector is a pPICZα A, pPIC9, pPIC9K, pHIL-S1, or pYAM75P vector. In one embodiment, the expression vector comprises a polynucleotide comprising a nucleotide sequence as shown in SEQ ID No. 2 or 3.

In another aspect, use of the yeast cell described herein for preparing collagen is provided.

In one embodiment, the collagen comprises repeating units of a sequence as shown in SEQ ID No. 1, the number of repeating units is 2-32, and the repeating units are directly linked. In one embodiment, the collagen comprises the amino acid sequence shown in SEQ ID No. 2 or 3. In one embodiment, the collagen is a recombinant type III humanized collagen. In one embodiment, the collagen is collagen having a 164.88° triple helix structure.

In another aspect, a method for producing collagen by fermentation is provided, comprising culturing the yeast cells described herein under a suitable condition, adding methanol to the yeast cells to induce expression, and harvesting the culture supernatant to obtain collagen.

In one embodiment, the culture medium is a BMMY medium supplemented with a yeast nitrogen base without amino acids and biotin.

In one embodiment, the collagen comprises repeating units of a sequence as shown in SEQ ID No. 1, the number of repeating units is 2-32, and the repeating units are directly linked. In one embodiment, the collagen comprises the amino acid sequence shown in SEQ ID No. 2 or 3. In one embodiment, the collagen is a recombinant type III humanized collagen, preferably collagen having a 164.88° triple helix structure.

The advantages of the present disclosure include:
1. The inventors have discovered yeast cells suitable for expressing a recombinant humanized type III collagen, construction methods and production methods thereof;
2. The yeast cells of the present invention are *P. pastoris,* which has the following advantages. *P. pastoris* has a larger expression amount and is easy to be scaled up as compared to prokaryotic expression systems such as *Escherichia coli;*
3. The method of the present disclosure synthesizes collagen having a 164.88° triple helix structure *in vitro,* achieving large-scale production at the ton level, and has important industrial significance;
4. The present disclosure does not require the step of eliminating endotoxins.

### Brief Description of the Drawings

Fig. 1 shows the electrophoresis of induction expression of P-012 with a high molecular weight.
Fig. 2 shows the electrophoresis of induction expression of P-C3T8 with a medium molecular weight.
Fig. 3 shows the cell adhesion activity of P-012 with a high molecular weight.
Fig. 4 shows the cell adhesion activity of P-C3T8 with a medium molecular weight.
Fig. 5 shows the circular dichroism results of P-012 with a high molecular weight.
Fig. 6 shows the circular dichroism results of P-C3T8 with a medium molecular weight.

### Detailed Description

In order to make the purpose, technical solutions and advantages of the present disclosure clearer, the technical solutions in the embodiments of the present invention will be clearly and completely described below in combination with the examples of the present invention. Obviously, the described examples are part of the embodiments of the present invention, not all of the embodiments. Based on the embodiments of the present disclosure, all other embodiments obtained by ordinary technicians in the field without creative work are within the protection scope of the present disclosure.

As used herein, a recombinant collagen is a new type of biomaterial having an identical or similar amino acid sequence as human collagen, which is screened and prepared with a gene encoding the functional region of human specific collagen as a template by using cutting-edge technologies such as structural biology and genetic engineering.

As used herein, "human recombinant type III collagen" refers to a recombinant protein consisting of or substantially consisting of a sequence derived from human type III collagen. Herein, human recombinant type III collagen can consist of or substantially consist of a fragment derived from human type III collagen or multiple repeats of the fragment. As used herein, "yeast" includes ascosporogenous yeast (Endomycetales), basidiosporogenous yeast, and yeast belonging to Fungi Imperfecti (Blastomycetes).

As used herein, yeast host cell means any yeast host cell that is susceptible to transformation, transfection, transduction, etc. with a nucleic acid construct or recombinant expression vector comprising a polynucleotide of the present disclosure. The yeast host cell may be a cell of *Candida, Hansenula, Kluyveromyces, Pichia, Saccharomyces, Schizosaccharomyces* or *Yarrowia,* such as a cell of *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis* or *Yarrowia lipolytica.* In the present application, the preferred yeast cell is *Pichia pastoris.* More preferably, *Pichia pastoris* is *Pichia pastoris* X33, GS115, SMD1168, KM71 or KM71H strain.

As used herein, the term "expression" includes any step involved in the production of a polypeptide, including but not limited to: transcription, post-transcriptional modification, translation, post-translational modification, and secretion.

As used herein, the term "expression vector" means a linear or circular DNA molecule that contains a polynucleotide encoding a polypeptide and is operably linked to a control sequence which is provided for its expression. In the present application, the expression vector is a yeast expression vector, preferably a pPICZα A, pPIC9, pPIC9K, pHIL-S1, or pYAM75P vector.

As used herein, the term "recombinant expression vector" means a single-stranded or double-stranded nucleic acid molecule which is isolated from a naturally occurring gene, or is modified in a manner that does not exist in nature to contain a nucleic acid segment, or is synthetic, and which contains one or more control sequences.

As used herein, the term "control sequence" means a nucleic acid sequence necessary for the expression of a polynucleotide encoding a mature polypeptide of the present disclosure. Each control sequence may be native (i.e., from the same gene) or foreign (i.e., from a different gene) to the polynucleotide encoding the polypeptide, or may be native or foreign to each other. Such control sequences include, but are not limited to, leader sequences, polyadenylation sequences, propeptide sequences, promoters, signal peptide sequences, and transcription terminators. At a minimum, the control sequences include promoters, and transcription and translation stop signals. These control sequences may be provided with multiple linkers for the purpose of introducing specific restriction sites that facilitate ligation of the control sequences to the coding region of the polynucleotide encoding the polypeptide.

### Collagen

The present disclosure provides collagen. The collagen may comprise repeating units of a sequence as shown in SEQ ID No. 1 or a sequence having a sequence identity of 80-100% (e.g., 80%, 85%, 86%, 87%, 88%, 89%, 90%, 91%, 92%, 93%, 94%, 95%, 96%, 97%, 98%, 99% or 100%) to the sequence as shown in SEQ ID No. 1. The number of repeating units is 2-32, such as 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 or 32. The individual repeating units may be directly linked. The collagen is a recombinant type III humanized collagen, preferably collagen having a 164.88° triple helix structure. The collagen may comprise an amino acid sequence as shown in SEQ ID No. 2 or 3, or an amino acid sequence having a sequence identity of 80-100% (e.g. 80%, 85%, 86%, 87%, 88%, 89%, 90%, 91%, 92%, 93%, 94%, 95%, 96%, 97%, 98%, 99% or 100%) to the amino acid sequence as shown in SEQ ID No. 2 or 3. The collagen described herein may have a triple helix structure or three identical chains (i.e., in trimer form). The sequence of each chain may be the sequence described herein.

### Recombinant expression vector

The present disclosure also relates to recombinant expression vectors comprising the nucleic acid of the present disclosure operably linked to one or more control sequences that direct expression of a coding sequence in a suitable host cell under conditions compatible with the control sequences. The vector may comprise a recombinant expression vector. The nucleic acid of the present disclosure may comprise the nucleotide sequence of SEQ ID NO. 4 or 5.

The nucleic acid may be manipulated in a variety of ways to provide expression of collagen. Depending on the expression vector, manipulation of the nucleic acid prior to its insertion into the vector may be desirable or necessary. Techniques for modifying nucleic acids using recombinant DNA methods are well known in the art.

The control sequence may be a promoter, i.e., a polynucleotide that is recognized by a host cell for expression of the polynucleotide encoding the collagen protein or polypeptide of the present disclosure. The promoter comprises a transcriptional control sequence that mediates expression of the collagen or polypeptide. The promoter may be any nucleic acid that exhibits transcriptional activity in a host cell, including variants, truncated and hybrid promoters, and may be obtained from a gene encoding an extracellular or intracellular collagen or polypeptide that is homologous or heterologous to the host cell.

Examples of suitable promoters for directing transcription of the vector or recombinant expression vector of the present disclosure in yeast host cells are not particularly limited. In yeast hosts, useful promoters are obtained from the following genes: *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triosephosphate isomerase (TPI), *Saccharomyces cerevisiae* metallothionein (CUP1), and *Saccharomyces cerevisiae 3-*phosphoglycerate kinase.

The control sequence may also be a transcription terminator recognized by the host cell to terminate transcription. The terminator may be operably linked to the 3' end of the polynucleotide encoding the collagen or polypeptide. Any terminator that is functional in the host cell may be used in the present disclosure. Preferred terminators for yeast host cells are obtained from the following genes: *Saccharomyces cerevisiae* enolase, *Saccharomyces cerevisiae* cytochrome C (CYC1), and *Saccharomyces cerevisiae* glyceraldehyde-3-phosphate dehydrogenase. The control sequence may also be an mRNA stabilizer region downstream of the promoter and upstream of the coding sequence of the gene, which increases the expression of the gene.

Examples of suitable mRNA stabilizer regions are obtained from the following genes: *Bacillus thuringiensis cryIIIA* gene (WO 94/25612) and *Bacillus subtilis* SP82 gene (Hue et al., 1995, Journal of Bacteriology 177: 3465-3471).

The control sequence may also be a leader sequence, i.e., a non-translated region of an mRNA that is important for translation of the host cell. The leader sequence is operably linked to the 5' end of the polynucleotide encoding the collagen or polypeptide. Any leader sequence that is functional in the host cell may be used. Suitable leader sequences for yeast host cells are obtained from the following genes: *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3' terminus of the polynucleotide and which, when transcribed, is recognized by the host cell as a signal to add polyadenylate residues to the transcribed mRNA. Any polyadenylation sequence that is functional in the host cell may be used. Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of the collagen and directs the collagen into the secretory pathway of the cell. The 5'-end of the coding sequence of the polynucleotide may itself contain a signal peptide coding sequence that is naturally linked in open reading frame to the segment of the coding sequence encoding the collagen. Alternatively, the 5'-end of the coding sequence may contain a signal peptide coding sequence that is foreign to the coding sequence. In cases where the coding sequence does not naturally contain a signal peptide coding sequence, a foreign signal peptide coding sequence may be required. Alternatively, the foreign signal peptide coding sequence may simply replace the natural signal peptide coding sequence in order to enhance the secretion of the collagen or polypeptide. However, any signal peptide coding sequence that directs the expressed collagen into the secretory pathway of the host cell may be used. Useful signal peptides for yeast host cells are obtained from the following genes: *Saccharomyces cerevisiae* alpha-factor and *Saccharomyces cerevisiae* invertase. Yeast cells may be used to prepare the collagen described herein.

### Host Cell

The present disclosure also relates to recombinant host cells comprising a polynucleotide of the present disclosure operably linked to one or more control sequences that direct the production of the collagen of the present disclosure. The construct comprising the polynucleotide is introduced into the host cell such that the construct is maintained as a chromosomal integrant or as an autonomously replicating extrachromosomal vector. The term "host cell" encompasses any progeny of a parent cell that is not identical to the parent cell due to mutations that arise during replication. The choice of host cell will depend largely on the gene encoding the collagen or polypeptide and its source.

Herein, the host cell may be a yeast cell, including ascosporogenous yeast (*Endomycetales*), basidiosporogenous yeast, and yeast belonging to *Fungi Imperfecti* (*Blastomycetes*). The yeast host cell may be a cell of *Candida, Hansenula, Kluyveromyces, Pichia, Saccharomyces, Schizosaccharomyces* or *Yarrowia,* such as a cell of *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis* or *Yarrowia lipolytica.* In particular, the present inventors have found that *Pichia pastoris* is suitable for producing the collagen described herein. Yeast cells can express the collagen described herein. Preferably, the collagen is recombinant type **III** humanized collagen, preferably collagen having a 164.88° triple helix structure. The yeast cell is *Pichia pastoris;* preferably *Pichia pastoris* X33, GS115, SMD1168, KM71 or KM71H strain. The yeast cell may contain a yeast expression vector, preferably a pPICZα A, pPIC9, pPIC9K, pHIL-S1, or pYAM75P vector. The expression vector can contain a polynucleotide comprising the nucleotide sequence as shown in SEQ ID No. 4 or 5.

### Construction method

The present disclosure provides a method for constructing a yeast cell expressing collagen, comprising introducing a recombinant expression vector into a yeast cell, wherein the recombinant expression vector comprises a polynucleotide encoding the collagen described herein. The recombinant expression vector can comprise a polynucleotide comprising the nucleotide sequence as shown in SEQ ID No. 4 or 5. The recombinant expression vector can be a yeast expression vector, preferably a pPICZα A, pPIC9, pPIC9K, pHIL-S1, or pYAM75P vector. The yeast can be *Pichia pastoris,* preferably *Pichia pastoris* X33, GS115, SMD1168, KM71, or KM71H strain.

The method can include one or more of the following steps:
(1) inserting a polynucleotide into an expression vector, preferably between NotI and XhoI restriction sites of pPiczalαA expression vector, to obtain a recombinant expression plasmid;
(2) enzymatically digesting the recombinant expression plasmid, preferably with sac1;
(3) purifying the enzymatically digested recombinant expression plasmid;
(4) introducing the enzymatically digested recombinant expression plasmid into a yeast cell by electroporation.

### Fermentation method

The present disclosure provides a method for producing collagen by fermentation. The method may include culturing a yeast cell described herein under a suitable condition, adding methanol to the yeast cell to induce expression, and harvesting the culture supernatant to obtain collagen. The conditions and culture medium for culturing yeast cells are known to those skilled in the art and are not particularly limited. For example, the culture medium may be a BMMY culture medium supplemented with a yeast nitrogen base without amino acids and biotin.

The fermentation method may include (1) culturing seed liquid, wherein the seed liquid is the seed liquid of the yeast cell described herein; (2) fermentation culture: inoculating the seed liquid into the fermentation medium, culturing the medium in a fermenter, and starting to add methanol in a batch fed course at a suitable growth stage to induce expression; (3) fermentation supernatant preparation: culturing the medium until a suitable time and terminating the culturing, taking the fermentation liquid and removing the yeast cells, filtering the fermentation liquid, and obtaining the fermentation supernatant.

The fermentation method may also include a step of purifying the collagen. The purification step is known to those skilled in the art and is not particularly limited. For example, the purification method may adopt the purification step described in CN109593126A.

The present invention is further illustrated by the following examples. It should be understood that the scope of the present invention shall be subject to the attached claims, and the examples shall not be interpreted as limiting the scope of the present disclosure.

### Examples

The present disclosure is further illustrated by the following examples, but any example or combination thereof should not be construed as limiting the scope or implementation of the present disclosure. The scope of the present disclosure is defined by the following claims. By combining this specification with common knowledge in the art, a person skilled in the art can clearly understand the scope defined by the claims. Without departing from the spirit and scope of the present disclosure, those skilled in the art can make any modifications or changes to the technical solution of the present disclosure, and such modifications and changes also fall within the scope of the present disclosure.

General methods of PCR, cloning, ligation, etc. of nucleotides are well known to those skilled in the art and can be found, for example, in the following literature: "Molecular cloning: A laboratory manual", Sambrook et al. (1989), Cold Spring Harbor lab., Cold Spring Harbor, New York; Ausubel, F. M. et al. (eds.); "Current protocols in Molecular Biology", John Wiley and Sons (1995); Harwood, C. R. and Cutting, S. M. (eds.); "DNA Cloning: A Practical Approach, Volumes I and II", D. N. Glover, ed. (1985); "Oligonucleotide Synthesis", M. J. Gait, ed. (1984); "Nucleic Acid Hybridization", B. D. Hames & S. J. Higgins, ed. (1985); "A Practical Guide To Molecular Cloning", B. Perbal (1984).

### Example 1: Construction, expression and screening of protein fragments

1) the amino acid sequence of a recombinant humanized type III collagen having a high molecular weight: 
gergapgfrgpagpngipgekgpagergapgergapgfrgpagpngipgekgpagergapgergapgfrg
pagpngipgekgpagergapgergapgfrgpagpngipgekgpagergapgergapgfrgpagpngipgek
gpagergapgergapgfrgpagpngipgekgpagergapgergapgfrgpagpngipgekgpagergapger
gapgfrgpagpngipgekgpagergapgergapgfrgpagpngipgekgpagergapgergapgfrgpagp
ngipgekgpagergapgergapgfrgpagpngipgekgpagergapgergapgfrgpagpngipgekgpag
ergapgergapgfrgpagpngipgekgpagergapgergapgfrgpagpngipgekgpagergapgergapg
frgpagpngipgekgpagergapgergapgfrgpagpngipgekgpagergap (SEQ ID No. 2;
 repeating unit is gergapgfrgpagpngipgekgpagergap; SEQ ID No. 1).
   Nucleotide sequence of a recombinant type III humanized collagen protein having a high molecular weight: 
aggtgaaagaggtgcc ccaggtttta gaggtccagc aggtccaaat ggtattccag gagagaaagg tccagcagga
gaaagaggtg cccccggtga gagaggtgct cccggattca gaggtcctgc cggtccaaat ggtattcccg gtgaaaaggg
acctgcagga gagagaggtg ccccaggaga gagaggagca ccaggattca gaggtccagc tggtccaaat ggtatccctg
gagagaaggg acccgctgga gaaagaggtg cccctggaga aagaggtgcc cccggtttta gaggacctgc tggacccaac
ggaatcccag gagagaaggg tcccgcaggt gagagaggtg ctcctggaga gagaggagca cctggattca gaggacctgc
aggacccaac ggaatacctg gtgaaaaagg tcctgccggt gaaagaggtg ctccaggtga aagaggagca ccaggtttta
gaggaccagc tggtcctaac ggtatccctg gtgaaaaagg acccgctggt gaaagaggag ccccaggtga gagaggtgct
cccggtttta gaggtccagc aggtccaaac ggaatacccg gtgaaaaagg acctgctgga gagagaggtg ctcctggaga
aagaggtgct cctggtttca gaggtccagc aggacccaat ggaatcccag gagaaaaagg acctgcaggt gaaagaggag
cccctggtga aagaggagca cctggtttta gaggaccagc aggtcctaat ggtattccag gagagaaggg acccgccgga
gaaagaggag cacccggtga aagaggagca ccaggtttca gaggaccagc tggaccaaac ggtattcccg gtgaaaaagg
accagctgga gagagaggtg caccaggaga aagaggtgct cccggtttca gaggtccagc cggaccaaat ggtatacctg
gagaaaaggg tccagcagga gaaagaggtg cacccggtga aagaggtgca ccaggtttta gaggtcccgc cggtccaaat
ggaatccctg gtgaaaaggg acccgctggt gaaagaggtg ctccaggaga gagaggagcc cccggtttta gaggtcccgc
 
tggtccaaat ggaatacctg gagagaaggg tcctgctggt gagagaggtg cacctggaga gagaggtgcc ccaggtttca
gaggacctgc cggtcccaat ggaatacccg gagaaaaagg tcccgcagga gagagaggtg ccccaggtga aagaggtgca
cccggtttca gaggacccgc cggtcctaat ggtatacctg gagaaaaagg accagccggt gagagaggtg ctcccggaga
gagaggagcc cctggtttca gaggtccagc aggaccaaac ggtattccag gagaaaaggg accagcagga gagagaggag
ccccatg (SEQ ID No .4)
2) the amino acid sequence of a recombinant type III humanized collagen protein having a medium molecular weight:

Gergapgfrgpagpngipgekgpagergapgergapgfrgpagpngipgekgpagergapgergapgfr
gpagpngipgekgpagergapgergapgfrgpagpngipgekgpagergapgergapgfrgpagpngipge
kgpagergapgergapgfrgpagpngipgekgpagergapgergapgfrgpagpngipgekgpagergapg
ergapgfrgpagpngipgekgpagergap (SEQ ID No .3)

The nucleotide sequence of the recombinant type III humanized collagen protein having the medium molecular weight:

ggcgagagaggggcaccgggttttcgaggtcctgccggacccaatggtattccaggggagaaaggccccgctggggaacggggag
cgcctggagagcgaggtgccccggggtttcgtggtccggcaggtccaaatggcatccctggcgaaaagggaccagccggcgagag
gggtgcacccggagaaaggggtgctccgggcttccgcggacccgccggtccgaacggaattcctggggagaaggggccagcggg
cgaaagaggtgctcctggggagaggggcgcgccaggattccggggtcctgctgggccgaacggcatacccggagagaaaggccca
gcaggggagcgaggagcacccggcgaaagggggggcgccaggatttcgcggacccgccggccccaacggtatcccgggggaaaa
agggcctgcgggtgaaagaggggccccgggcgaacgtggggcacccggtttccgaggaccagcgggccctaacggaatacctggt
gagaagggtccagctggggagcggggtgcgccgggggaaagaggtgcacccggcttccgtgggccagccggccctaatggtatcc
ccggcgaaaaggggcctgcaggtgaacgtggggctcctggggagcgcggcgctcccggatttcggggcccggctggaccaaatgg
aattcctggagagaaaggcccggccggtgaacgcggagcgccg (SEQ ID No .5)

The above encoding nucleotide sequence is commercially synthesized. The above coding nucleotide sequence was inserted between the NotI and XhoI restriction sites of the pPiczalαA expression vector, with the target gene at the end of the signal cleavage, to obtain the recombinant expression plasmids P-012 (having high molecular weight) and P-C3T8 (having medium molecular weight).

### Example 2: Engineering of a yeast for the recombinant type III humanized collagen

1. Enzymatic digestion of a plasmid
   20-40 µg of each of the successfully constructed expression plasmids P-012 and P-C3T8 were taken respectively, enzymatically digested with sac1, and enzymatically digested at 37°C for 2-3 hours according to the enzymatic digestion ratio for the restriction enzyme. After the enzymatic digestion, a gel electrophoresis for the nucleic acid was performed to detect the plasmid before and after enzymatic digestion to verify whether it is completely enzymatically digested and the position of the band after digestion is greater than that before digestion.
2. Plasmid purification
   The enzymatically digested plasmid was purified with a plasmid purification kit to remove impurities such as ions, dissolved with 20-25 µl of sterilized purified water. The concentration of plasmid was detected, and the plasmid was placed at 4°C for use.
3. Engineering of a yeast cell
   The yeast competent cells (X33) were taken out and the following operations were performed under the biosafety cabinet:
   a. A 0.2cm electroporation cup was taken, soaked in 75% alcohol for 10 minutes for disinfection, rinsed repeatedly with sterile water 3 times and dried;
   b. The electroporation cup was placed in an ice bath for 20 minutes and the electroporator was turned on;
   c. 80 µl of competent cells and 5-15 µg of the enzymatically digested plasmid were added to the sterilized 1.5 ml EP tube, and mixed repeatedly with a pipette;
   d. 100 µl of the mixed solution was transferred to the electroporation cup. After ice bathing for 5 minutes, the outer surface of the electroporation cup was wiped, the electroporation cup was transferred to the electroporator slot, and electroporation was performed by clicking start;
   e. After the electroporation was completed, 1 ml of 1 M sterile sorbitol solution was added to the electroporation cup, sealed with a sealing film, and incubated at 30°C for 2h;
   f. The mixed solution in the electroporation cup was transferred to a sterilized 10 ml EP tube, then added with 1 ml of YPD liquid medium (yeast peptone 0.2 g/L, yeast extract powder 0.1 g/L) in the EP tube and cultured at 30°C, 300rpm for 1 hour.
   g. Spreading a plate: Under the biosafety cabinet, 200 µl of the transformed and incubated yeast cell solution was taken and added to the plates with the poured medium numbered 1-3 and a plate numbered 4 as the control. The plates were evenly spread with a sterile spreading rod, and then cultured at 30°C for about 2-5 days.
   h. Strain culture: the plates were cultured until bacteria plaques grown out. 10 ml of a solution of anhydrous glucose was added to 40 ml of YPD liquid medium, and when the temperature of the medium was dropped to room temperature, 50 ml of the medium was dispensed into ten 50 ml bioreaction tubes at 5 ml/tube, added with bleomycin solution at a concentration of 2000 mg/L. 10 single colonies on the plates were picked up and inoculated into 10 small tubes for overnight culture at 30°C and 300 rpm. 200 ml of BMGY culture medium was prepared and sterilized in the shake flask, then 20 ml of 10*YNB stock solution and 200 µl of 500*biotin stock solution were added. The shake flask was mixed well. 200 ml of culture medium was distributed into ten 50 ml centrifuge tubes with 20 ml/tube. Then 4 ml of the cultured yeast cell solution was taken and inoculated into 10 centrifuge tubes with 20 ml/tube respectively. The medium was cultured overnight until the OD value of the yeast cell solution was between 3-10.
   i. Induction of expression: 500 ml BMMY culture medium was prepared and sterilized in the shake flask before being added with 50 ml 10*YNB stock solution and 500 µl 500*biotin stock solution, and mixed well. 500 ml of culture medium was divided into ten 250 ml shake flasks with 50 ml/flask. The cultured yeast cell solution was centrifuged at 1500 rpm for 10 min and the supernatant was discarded. The yeast cell pellets in 10 centrifuge tubes were suspended with the culture medium in 10 shake flasks respectively and transferred to their corresponding shake flasks which were shaken at 30°C and 300rpm. 1 ml from each of the 10 shake flasks was sampled into 1.5 ml EP tubes every 24 hours and stored at -20°C. 1% methanol (filtered with a 0.22 µm filter) was added to the shake flasks to induce expression until the end of the 5th day. The samples in the 1.5 ml EP tubes were centrifuged at 12000 rpm for 5 min, and the supernatant was taken for detection by electrophoresis to observe the expressions of 10 colonies respectively.
4. Detection by Electrophoresis

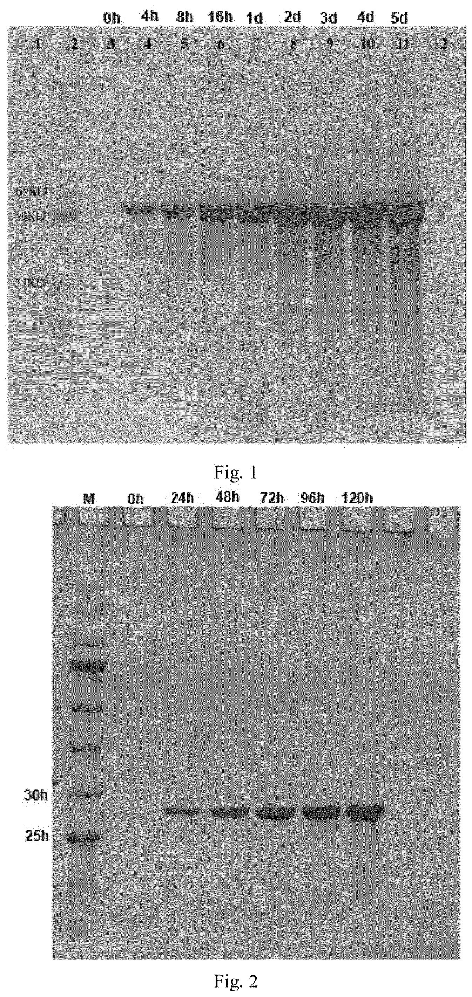

In particular, 40 µl of sample solution was taken, added with 10 µl 5x protein loading buffer (250 mM Tris-HCl (pH: 6.8), 10% SDS, 0.5% bromophenol blue, 50% glycerol, 5% β-mercaptoethanol), boiled in 100°C boiling water for 10 min, then added to SDS-PAGE protein gel at 10 µl per well, run at 80V for 2 hours. The SDS-PAGE protein gel was stained with Coomassie brilliant blue staining solution (0.1% Coomassie brilliant blue R-250, 25% isopropanol, 10% glacial acetic acid) for 20 min, and then de-stained with protein destaining solution (10% acetic acid, 5% ethanol). The detection results obtained by electrophoresis are shown in Figs. 1 and 2. The theoretical molecular weight of the P-012 target protein is 44.75KD, and the apparent molecular weight is 50KD. The theoretical molecular weight of the P-C3T8 target protein is 22.39KD, and the apparent molecular weight is 28KD. The detection results obtained by electrophoresis show that the yeast engineering was successful.

### Example 3: Mass spectrometry detection of recombinant type III humanized collagens

1. Experimental instruments:
1) High-resolution mass spectrometer: XevoG2-XS QTof (Waters)
2) Ultra-high performance liquid chromatography: UPLC (Acquity UPLC I-Class) (Waters)

2. Materials and reagents
1) Guanidine HCl (Sigma)
2) Urea (Bio-Rad)
3) Tris-base (Bio-Rad)
4) DTT (Bio-Rad)
5) IAM (Sigma)
6) Zeba Spin column (Pierce)
7) ACQUITY UPLC Peptide BEH C18 Column, 300Å, 1.7 µm, 2.1 mm X 150 mm (Waters)
8) UNIFI (Waters)
9) Trypsin (Promega)
10) Chymotrypsin (Sigma)
11) Glu-C (Wako)
12) LysC (Wako)

3. Experimental methods
1) Enzymatic digestion with trypsin, chymotrypsin and Glu-C: an appropriate amount of the test sample was taken and added with trypsin, chymotrypsin and Glu-C after appropriate pretreatment, and enzymatic digest was performed at 37°C for 20 hours.
2) High performance liquid chromatography: After enzymatic digestion, the test sample was separated by ultra-high performance liquid system Acquity UPLC I-Class. Liquid A of liquid phase is 0.1% FA aqueous solution, and Liquid B is 0.1% FA acetonitrile solution. The test sample was loaded onto the column by an automatic sampler and then separated by a chromatographic column. The column temperature was 55°C, the flow rate was 300 µl/min, and the TUV detector wavelength was 214 nm. The relevant liquid phase gradient was as follows:

| | Time/min | A/% | B% |
|---|---|---|---|
| 1 | 3 | 100 | 0 |
| 2 | 63 | 60 | 40 |
| 3 | 63.1 | 2 | 98 |
| 4 | 66 | 2 | 98 |
| 5 | 66.1 | 100 | 0 |
| 6 | 75 | 100 | 0 |

3) Identification by Mass spectrometry: After desalting and separation by ultra-high performance liquid chromatography were completed, the test sample was analyzed by mass spectrometry using a XevoG2-XS QTof mass spectrometer (Waters). Analysis time: 63 min, detection method: positive ion, MS, scanning range (m/z): 300-2000.
4) Processing of Mass spectrometry data: The raw data was queried by UNIFI (1.8.2, Waters) software, and the main parameters were as follows (Table 1):

| Item | Value |
|---|---|
| Enzyme | Trypsin, Chymotrypsin, Glu-C |
| modifications | Deamidated(NQ), Oxidation(M), Carbamidomethyl(C) |
| M/Z tolerance | ±15ppm |
| Fragment tolerance | Fragment tolerance |
| Database | 20200604366.fasta |
| Filter | Minimum fragment ions:3 |

4. Experimental results and analysis
After the test samples were enzymatically digested with Trypsin, Chymotrypsin, and Glu-C respectively in solution, the peptide samples were analyzed by LC-MS/MS equipment, and the raw data obtained was queried by UNIFI software.
1) The molecular weight of P-012 and the corresponding polypeptide detected by mass spectrometer.

| Observed value | Mr (Expected value) | Peptide |
|---|---|---|
| 1171.2805 | 1170.1811 | GERGAPGFRGPA |
| 1660.8642 | 1659.8569 | GPAGPNGIPGEKGPAGER |
| 1678.8994 | 1677.8921 | GAPGFRGPAGPNGIPGEK |
| 2244.0007 | 2242.9934 | GPAGPNGIPGEKGPAGERGAPGER |
| 2244.3176 | 2243.3104 | GPAGPNGIPGEKGPAGERGAPGER |
| 2246.2154 | 2245.2081 | GAPGFRGPAGPNGIPGEKGPAGER |

The coverage of the detected polypeptide fragment was 100%.
2) The molecular weight of P-C3T8 and the corresponding polypeptide detected by mass spectrometer.

| Observed value | Mr (Expected value) | Peptide |
|---|---|---|
| 1706.8815 | 1705.5321 | GERGAPGFRGPAGPNGIP |
| 1093.5637 | 1092.5564 | GPAGPNGIPGEK |
| 1660.8636 | 1659.8563 | GPAGPNGIPGEKGPAGER |
| 1678.8907 | 1677.8834 | GAPGFRGPAGPNGIPGEK |
| 2244.3176 | 2243.3104 | GPAGPNGIPGEKGPAGERGAPGER |
| 2246.1276 | 2245.1203 | GAPGFRGPAGPNGIPGEKGPAGER |

The coverage of the detected polypeptide fragment was 100%.

After the enzymatically digested samples were analyzed by LC-MS/MS, the query results were integrated. The peptide coverage of the final samples obtained was 100%, and the detection results were very reliable.

### Example 4: Detection of cell adhesion activity of recombinant humanized type III collagens

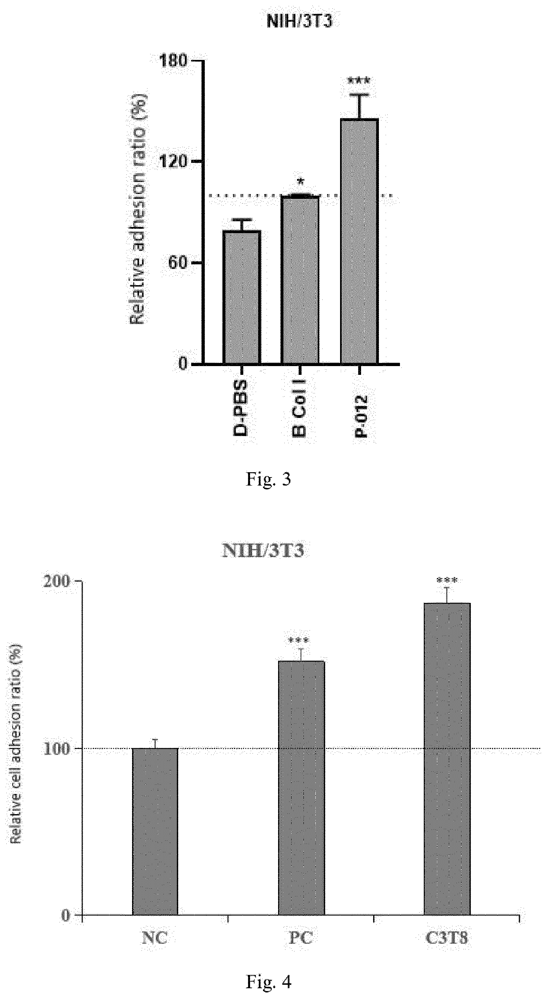

The method for detecting the activity of collagen can refer to the literature Juming Yao, Satoshi Yanagisawa, Tetsuo Asakura, Design, Expression and Characterization of Collagen-Like Proteins Based on the Cell Adhesive and Crosslinking Sequences Derived from Native Collagens, J Biochem. 136, 643-649 (2004). The specific implementation method was as follows:
(1) The concentration of the protein sample to be tested, including bovine type I collagen (China Food and Drug Inspection Institute, No.: 380002) and the recombinant humanized protein provided by the present disclosure was detected by ultraviolet absorption method. Specifically, the ultraviolet absorption of the sample at 215 nm and 225 nm was measured respectively, and the protein concentration was calculated using the empirical formula C (µg/mL) = 144 x (A215-A225). Note that the detection needs to be performed when A215 < 1.5. The principle of this method is to measure the characteristic absorption of peptide bonds under far-ultraviolet light, which is not affected by the chromophore content. The interfering substances are less in the method. The method is easy to operate, and is suitable for detecting human collagen and its analogs that are not stained with Coomassie Brilliant Blue. (Reference: Walker JM. The Protein Protocols Handbook, second edition. Humana Press. 43-45.). After the protein concentration was detected, the concentration of all proteins to be tested was adjusted to 0.5 mg/mL with PBS.
(2) The 96-well plate was added with 100 µL of various protein solutions and blank PBS solution controls and left to stand at room temperature for 60 minutes.
(3) 105 NIH/3T3 well cultured cells were added to each well and incubated at 37°C for 60 minutes.
(4) Each well was washed with PBS 4 times.
(5) The absorbance at OD492nm was detected with the LDH detection kit (Roche, 04744926001). Based on the value of the blank control, the cell adhesion rate can be calculated. The calculation formula was as follows: Cell adhesion rate = x 100%. The cell adhesion rate can reflect the activity of collagen. The higher the activity of the protein, the better the external environment that can be provided to the cells in a short time, helping the cells to adhere to the wall. The determination results are shown in Figs. 3 and 4.

### Example 5: Circular dichroism UV scanning analysis of recombinant type III humanized collagens

Experimental methods
(1) Instrument parameter setting
   Band width: 1.0 nm
   Step: 1.0 nm
   Measurement range: 190-260 nm (far-UV scan)/250-340 nm (near-UV scan)
   Time-per-point: 0.5s
   Repeats: 3 times
   Cell length: 10 mm|0.5 mm
   Temperature: room temperature
(2) Near and far UV scans of standard
   The scanning wavelength was set to 180-340 nm for background test and blank buffer test, and then the near and far UV absorptions of circular dichroism of 1 mg/mL CSA standard solution was collected in the range of 180-340 nm.
(3) Sample processing
   P-012 and P-C3T8 protein samples were taken and the proteins were concentrated to a protein concentration of 1 mg/ml with 10KD ultrafiltration concentrators (Millipore).
(4) Far UV scan of samples
   The cuvette was soaked in 2M HNO₃ overnight, rinsed with deionized water and dried. The data of the background were collected firstly, then the data of the blank buffer were collected, then an appropriate amount of the test samples was added to the cuvette, a far UV scan was performed at 190-260 nm according to the above parameters and data were collected.
(5) Near UV scan of samples
   The cuvette was soaked in 2M HNO₃ overnight, rinsed with deionized water and dried. The data of background were collected firstly, then the data of blank buffer were collected, then an appropriate amount of the test samples were added to the cuvette, a near UV scan was performed at 250-340 nm according to the above parameters and data were collected.
(6) Scanning spectrum processing
   Subtract baseline and smoothing treatments are performed on all scanned spectra using the Pro-Data Viewer software.

### Experimental results and analysis

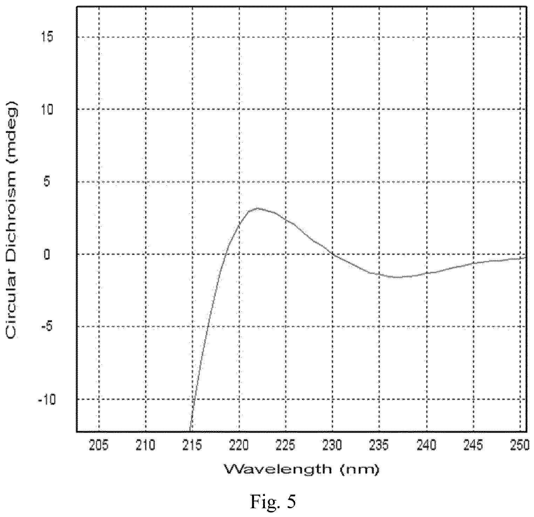

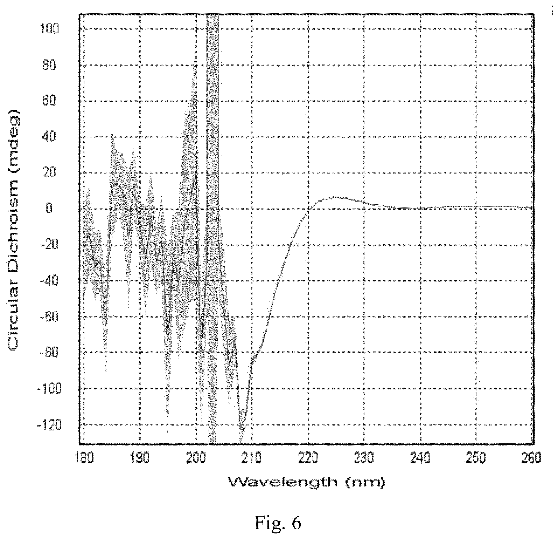

The results showed that both P-012 and P-C3T8 had positive peaks at 221 nm, indicating that both of the proteins have triple helix structures. The results are shown in Figs. 5 and 6.

## Claims

1. A method for constructing a yeast cell expressing collagen, comprising introducing a recombinant expression vector into the yeast cell, wherein the recombinant expression vector comprises a polynucleotide encoding the collagen, wherein the collagen comprises repeating units of the sequence as shown in SEQ ID No. 1, the number of the repeating units is 2-32, and the repeating units are directly linked; preferably, wherein the collagen is a recombinant type III humanized collagen, preferably collagen having a 164.88° triple helix structure.

2. The method according to claim 1, wherein the collagen comprises an amino acid sequence as shown in SEQ ID No. 2 or 3; preferably, the polynucleotide comprises a nucleotide sequence as shown in SEQ ID No. 4 or 5.

3. The method according to claim 1 or 2, wherein the recombinant expression vector is a yeast expression vector, preferably a pPICZα A, pPIC9, pPIC9K, pHIL-S1, or pYAM75P vector.

4. The method according to any one of claims 1-3, wherein the yeast is *Pichia pastoris;* preferably *Pichia pastoris* X33, GS115, SMD1168, KM71 or KM71H strain.

5. The method according to any one of claims 1-4, comprising one or more of the following steps:
(1) inserting the polynucleotide into an expression vector, preferably between NotI and XhoI restriction sites of pPiczalαA expression vector, to obtain a recombinant expression plasmid;
(2) enzymatically digesting the recombinant expression plasmid, preferably with sac1;
(3) purifying the enzymatically digested recombinant expression plasmid;
(4) introducing the enzymatically digested recombinant expression plasmid into the yeast cell by electroporation.

6. A yeast cell expressing collagen, wherein the collagen comprises repeating units of the sequence as shown in SEQ ID No. 1, the number of repeating units is 2-32, and the repeating units are directly linked; the yeast cell is *Pichia pastoris;* preferably *Pichia pastoris* X33, GS115, SMD1168, KM71 or KM71H strain; preferably, wherein the collagen comprises the amino acid sequence as shown in SEQ ID No. 2 or 3; preferably, wherein the collagen is a recombinant type III humanized collagen, preferably a collagen having a 164.88° triple helix structure.

7. The yeast cell according to claim 6, comprising a yeast expression vector, preferably pPICZα A, pPIC9, pPIC9K, pHIL-S1, or pYAM75P vector; wherein the expression vector comprises a polynucleotide comprising the nucleotide sequence as shown in SEQ ID No. 4 or 5.

8. Use of the yeast cell according to claim 6 or 7 for preparing collagen, preferably, wherein the collagen comprises repeating units of the sequence as shown in SEQ ID No. 1, the number of the repeating units is 2-32, and the repeating units are directly linked; preferably, wherein the collagen comprises the amino acid sequence as shown in SEQ ID No. 2 or 3; preferably, the collagen is a recombinant type III humanized collagen, preferably collagen having a 164.88° triple helix structure.

9. A method for producing collagen by fermentation, comprising culturing the yeast cell according to claim 6 or 7 under a suitable condition, adding methanol to the yeast cell to induce expression, and harvesting the culture supernatant to obtain the collagen; preferably, wherein the culture medium is a BMMY medium supplemented with biotin and a yeast nitrogen base without amino acids.

10. The method according to claim 9, comprising:
(1) culturing a seed liquid, wherein the seed liquid is the seed liquid of the yeast cell of claim 6 or 7;
(2) fermentation culture: inoculating the seed liquid into a fermentation medium, culturing the medium in a fermenter, and starting to add methanol in a batch-fed course at a suitable growth stage to induce expression;
(3) preparation of a fermentation supernatant: culturing the medium until a suitable time and terminating the culturing, taking the fermentation liquid and removing the yeast cells, filtering the fermentation liquid, and obtaining the fermentation supernatant;
preferably, wherein the collagen comprises repeating units of the sequence as shown in SEQ ID No. 1, the number of the repeating units is 2-32, and the repeating units are directly linked; preferably, wherein the collagen comprises the amino acid sequence as shown in SEQ ID No. 2 or 3; preferably, the collagen is recombinant type III humanized collagen, preferably collagen having a 164.88° triple helix structure.
